# EUROPÄISCHE PATENTSCHRIFT

(11) **EP 4 197 997 B1**
(45) Veröffentlichungstag und Bekanntmachung des Hinweises auf die Patenterteilung: **15.05.2024**
(21) Anmeldenummer: 21215342.3
(22) Anmeldetag: 17.12.2021
(51) Int. Cl.: C07C 45/00, C07F 15/00

(54) **PT-IOD-KOMPLEX UND PT-BROM-KOMPLEX MIT XANTPHOSDERIVATEN**
PT-IODINE COMPLEX AND PT-BROMINE COMPLEX WITH XANTPHOS DERIVATIVES
COMPLEXE PT-IODE ET COMPLEXE PT-BROME COMPORTANT DES XANTPHOSDERIVÉS

(43) Veröffentlichungstag der Anmeldung: 21.06.2023
(73) Patentinhaber: Evonik Oxeno GmbH & Co. KG, 45772 Marl (DE)
(72) Erfinder: SCHNEIDER, Carolin, 40789 Monheim am Rhein (DE); JACKSTELL, Ralf, 18106 Rostock (DE); BELLER, Matthias, 18211 Ostseebad Nienhagen (DE); FRANKE, Robert, 45772 Marl (DE)
(74) Vertreter: Evonik Patent Association

(56) Entgegenhaltungen:
- BRONGER R P J ET AL: "INFLUENCE OF THE BITE ANGLE ON THE HYDROFORMYLATION OF INTERNAL OLEFINS TO LINEAR ALDEHYDES", ORGANOMETALLICS, AMERICAN CHEMICAL SOCIETY, Bd. 22, Nr. 25, 8. Dezember 2003 (2003-12-08), Seiten 5358-5369, XP001209898, ISSN: 0276-7333, DOI: 10.1021/OM034012F

## Beschreibung

Die vorliegende Erfindung betrifft einen Pt-lod-Komplex und einen Pt-Brom-Komplex mit Xantphosderivaten , sowie deren Verwendung zur Katalyse einer Hydroformylierungsreaktion.

In C. Botteghi et al., Journal of Molecular Catalysis A: Chemical 200, (2003), 147-156 wird der Einsatz von Pt(Xantphos)Cl₂ zur Hydroformylierung von 2-Tosyloxystyrol beschrieben.

In R.P.J. Bronger et al., Organometallics 2003, 22, 5358-5369, "Influence of the Bite Angle on the Hydroformylation of Internal Olefins to Linear Aldehydes" werden eine Reihe von Liganden beschrieben und deren strukturellen Winkel betrachtet.

Der vorliegende Erfindung lag die Aufgabe zugrunde, einen neuen Komplex bereitzustellen. Der Komplex soll hierbei bei der Katalyse von Hydroformylierungsreaktionen gegenüber dem im Stand der Technik beschriebenen Komplex Pt mit Cl₂ eine gesteigerte Ausbeute liefern.

Diese Aufgabe wird gelöst durch einen Komplex gemäß Anspruch 1.

Komplex umfassen:
a) Pt;
b) einen Liganden entsprechend der Formel (I): 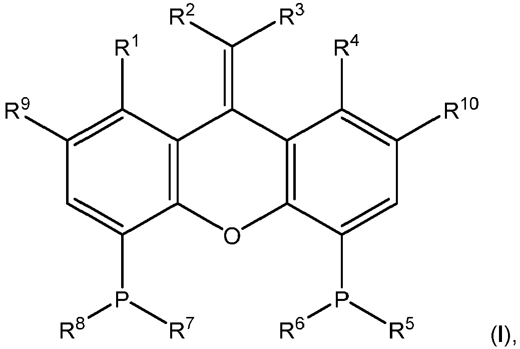 wobei R¹, R², R³, R⁴, R⁵, R⁶, R⁷, R⁸, R⁹, R¹⁰ ausgewählt sind aus: -H, -(C₁-C₁₂)-Alkyl, -(C₆-C₂₀)-Aryl, und R¹, R², R³, R⁴ kondensierte Ringsysteme ausbilden können;
c) einen lod-Liganden oder Brom-Liganden.

Der Begriff (C₁-C₁₂)-Alkyl umfasst geradkettige und verzweigte Alkylgruppen mit 1 bis 12 Kohlenstoffatomen. Vorzugsweise handelt es sich dabei um (C₁-C₈)-Alkylgruppen, besonders bevorzugt (C₁-C₆)-Alkyl, am meisten bevorzugt (C₁-C₄)-Alkyl.

Geeignete (C₁-C₁₂)-Alkylgruppen sind insbesondere Methyl, Ethyl, Propyl, Isopropyl, *n*-Butyl, *iso*-Butyl, *sec*-Butyl, *tert*-Butyl, *n*-Pentyl, 2-Pentyl, 2-Methylbutyl, 3-Methylbutyl, 1,2-Dimethylpropyl, 1,1-Dimethylpropyl, 2,2-Dimethylpropyl, 1-Ethylpropyl, n-Hexyl, 2-Hexyl, 2-Methylpentyl, 3-Methylpentyl, 4-Methylpentyl, 1,1-Dimethylbutyl, 1,2-Dimethylbutyl, 2,2-Dimethylbutyl, 1,3-Dimethylbutyl, 2,3-Dimethylbutyl, 3,3-Dimethylbutyl, 1,1,2-Trimethylpropyl, 1,2,2-Trimethylpropyl, 1-Ethylbutyl, 1-Ethyl-2-methylpropyl, n-Heptyl, 2-Heptyl, 3-Heptyl, 2-Ethylpentyl, 1-Propylbutyl, *n*-Octyl, 2-Ethylhexyl, 2-Propylheptyl, Nonyl, Decyl.

Der Begriff (C₆-C₂₀)-Aryl umfasst mono- oder polycyclische aromatische Kohlenwasserstoffreste mit 6 bis 20 Kohlenstoffatomen. Vorzugsweise handelt es sich dabei um (C₆-C₁₄)-Aryl, besonders bevorzugt (C₆-C₁₀)-Aryl.

Geeignete (C₆-C₂₀)-Arylgruppen sind insbesondere Phenyl, Naphthyl, Indenyl, Fluorenyl, Anthracenyl, Phenanthrenyl, Naphthacenyl, Chrysenyl, Pyrenyl, Coronenyl. Bevorzugte (C₆-C₂₀)-Arylgruppen sind Phenyl, Naphthyl und Antracenyl.

In einer Ausführungsform stehen R⁵, R⁶, R⁷, R⁸ für -(C₆-C₂₀)-Aryl.

In einer Ausführungsform stehen R⁵, R⁶, R⁷, R⁸ für -Ph.

In einer Ausführungsform stehen R⁹ und R¹⁰-H.

In einer Ausführungsform stehet mindestens einer der Reste R¹, R⁴ für -H.

In einer Ausführungsform stehen R¹, R⁴ für -H.

In einer Ausführungsform stehen R² und R³ für -(C₁-C₁₂)-Alkyl.

In einer Ausführungsform stehen R² und R³ für -CH₃.

In einer Ausführungsform weist der Ligand entsprechend der Formel (**I**) die Struktur (**1**) oder (**2**) auf:

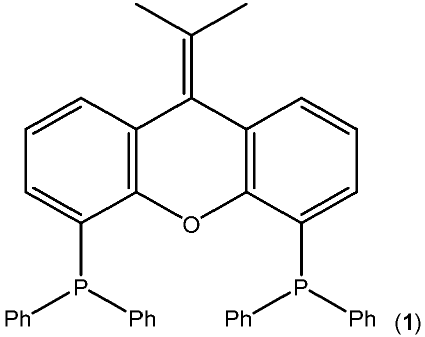

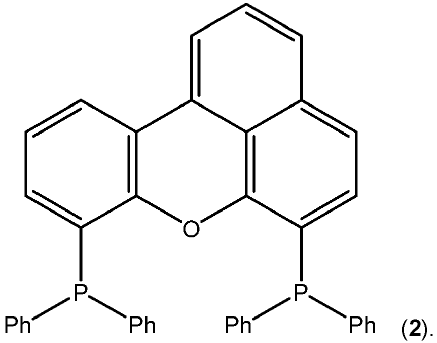

In einer Ausführungsform weist der Ligand entsprechend der Formel (**I**) die Struktur (**1**) auf:

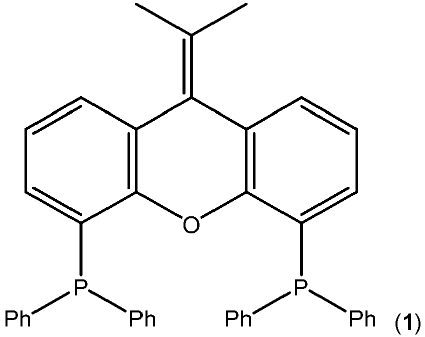

In einer Ausführungsform weist der Ligand entsprechend der Formel (**I**) die Struktur (**2**) auf:

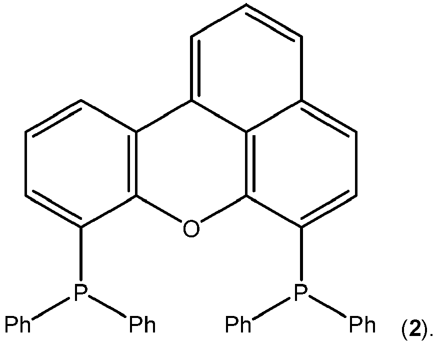

In einer Ausführungsform weist der Komplex genau einen Liganden entsprechend der Formel (**I**) auf.

In einer Ausführungsform weist der Komplex mindestens zwei lod-Liganden auf.

In einer Ausführungsform weist der Komplex genau zwei lod-Liganden auf.

In einer Ausführungsform weist der Komplex mindestens zwei Brom-Liganden auf.

In einer Ausführungsform weist der Komplex genau zwei Brom-Liganden auf.

Neben dem Komplex an sich, wird auch dessen Verwendung zur Katalyse einer Hydroformylierungsreaktion beansprucht.

Verwendung eines zuvor beschriebenen Komplexes zur Katalyse einer Hydroformylierungsreaktion.

Im Folgenden soll die Erfindung anhand von Ausführungsbeispielen näher erläutert werden.

### Versuchsbeschreibung

Ein Vial wurde mit PtX₂ (X = Halogen), Ligand und einem im Ofen getrockneten Rührstab bestückt. Dann wird das Vial mit Septum (PTFE-beschichteter StyrolButadien-Kautschuk) und Phenolharzdeckel verschlossen. Das Vial wird dreimal evakuiert und mit Argon wieder befüllt. Mit einer Spritze wurden Toluol und Olefin in das Vial gegeben. Das Vial wurde in eine Legierungsplatte gestellt, die in einen Autoklav (300 ml) der Reihe 4560 von Parr Instruments unter Argon Atmosphäre überführt wurde. Nach dreimaligem Spülen des Autoklaven mit CO/H₂ wurde der Synthesegasdruck auf 40 bar bei Raumtemperatur erhöht. Die Reaktion wurde 18 h bei 80 °C / 120 °C durchgeführt. Nach Beendigung der Reaktion wurde der Autoklav auf Raumtemperatur abgekühlt und vorsichtig entspannt. Der Ausbeute und Selektivität wurden durch GC-Analyse bestimmt.

### Hydroformylierung 1-Octen

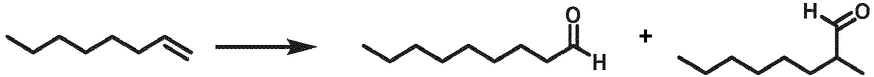

### Reaktionsbedingungen:

1.0 mmol 1-Octen, 0.5 mol% PtX₂, 2,0 Äquivalente Ligand, Lösungsmittel: Toluol, p(CO/H₂): 40 bar, T: 80 °C, t: 18 h.

### Ausbeuten:

| Ligand | Halogen | Ausbeute [%] |
|---|---|---|
| 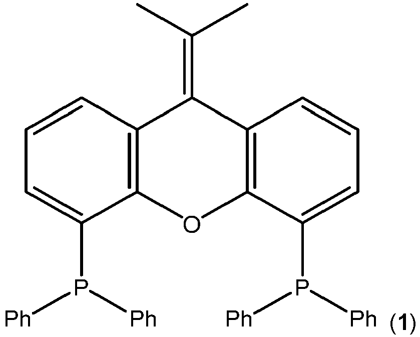 | I / Br / Cl | 98 / 65 / < 1 |

### Hydroformylierung 2-Octen

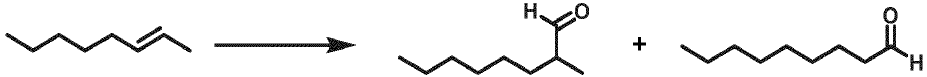

### Reaktionsbedingungen:

1.0 mmol 2-Octen, 0.5 mol% PtX₂, 2,0 Äquivalente Ligand, Lösungsmittel: Toluol, p(CO/H₂): 40 bar, T: 120 °C, t: 18 h.

### Ausbeuten:

| Ligand | Halogen | Ausbeute [%] |
|---|---|---|
| 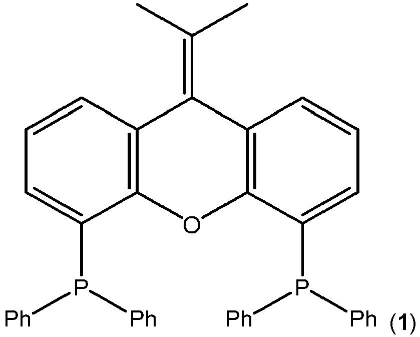 | I / Br/ Cl | 85 / 81 / <1 |
| 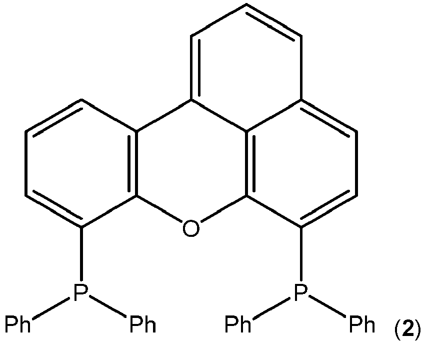 | I / Br/ Cl | 87 / 63 / <1 |

Wie die Versuchsergebnisse zeigen, wird die Aufgabe durch den erfindungsgemäßen Komplex gelöst.

## Patentansprüche

1. Komplex umfassen:
a) Pt;
b) einen Liganden entsprechend der Formel (**I**): 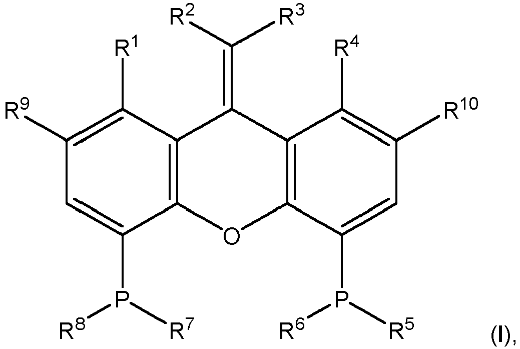 wobei R¹, R², R³, R⁴, R⁵, R⁶, R⁷, R⁸, R⁹, R¹⁰ ausgewählt sind aus: -H, -(C₁-C₁₂)-Alkyl, -(C₆-C₂₀)-Aryl, und R¹, R², R³, R⁴ kondensierte Ringsysteme ausbilden können;
c) einen lod-Liganden oder Brom-Liganden.

2. Komplex nach Anspruch 1,
wobei R⁵, R⁶, R⁷, R⁸ für -(C₆-C₂₀)-Aryl stehen.

3. Komplex nach einem der Ansprüche 1 oder 2,
wobei R⁵, R⁶, R⁷, R⁸ für -Ph stehen.

4. Komplex nach einem der Ansprüche 1 bis 3,
wobei R⁹ und R¹⁰ für -H stehen.

5. Komplex nach einem der Ansprüche 1 bis 4,
wobei mindestens einer der Reste R¹, R⁴ für -H steht.

6. Komplex nach einem der Ansprüche 1 bis 5,
wobei R² und R³ für -(C₁-C₁₂)-Alkyl stehen.

7. Komplex nach einem der Ansprüche 1 bis 6,
wobei der Ligand entsprechend der Formel (**I**) die Struktur (**1**) oder (**2**) aufweist:

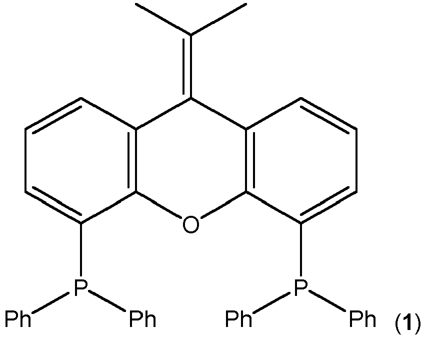

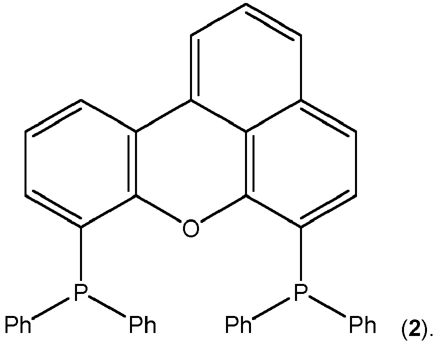

8. Komplex nach einem der Ansprüche 1 bis 7,
wobei der Ligand entsprechend der Formel (**I**) die Struktur (**1**) aufweist:

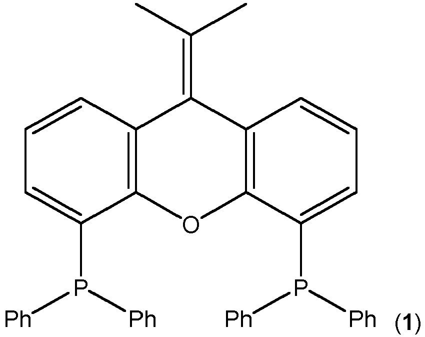

9. Komplex nach einem der Ansprüche 1 bis 7, wobei der Ligand entsprechend der Formel (**I**) die Struktur (**2**) aufweist:

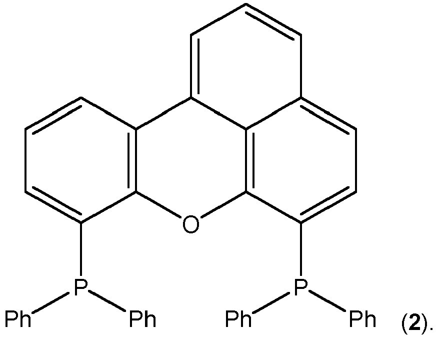

10. Komplex nach einem der Ansprüche 1 bis 9,
wobei der Komplex genau einen Liganden entsprechend der Formel (**I**) aufweist.

11. Komplex nach einem der Ansprüche 1 bis 10,
wobei der Komplex mindestens zwei lod-Liganden aufweist.

12. Komplex nach Anspruch 11,
wobei der Komplex genau zwei lod-Liganden aufweist.

13. Komplex nach einem der Ansprüche 1 bis 10,
wobei der Komplex mindestens zwei Brom-Liganden aufweist.

14. Komplex nach Anspruch 13,
wobei der Komplex genau zwei Brom-Liganden aufweist.

15. Verwendung eines Komplexes nach einem der Ansprüche 1 bis 14 zur Katalyse einer Hydroformylierungsreaktion.

## Claims

1. Complex comprising:
a) Pt;
b) a ligand corresponding to formula (I): 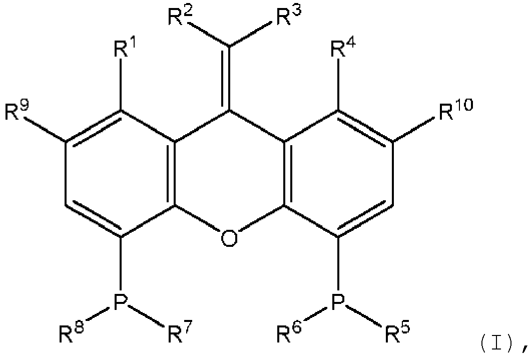 where R¹, R², R³, R⁴, R⁵, R⁶, R⁷, R⁸, R⁹, R¹⁰ are selected from: -H, -(C₁-C₁₂) -alkyl, -(C₆-C₂₀)-aryl, and R¹, R², R³, R⁴ may form fused ring systems;
c) an iodine ligand or bromine ligand.

2. Complex according to Claim 1,
wherein R⁵, R⁶, R⁷, R⁸ are -(C₆-C₂₀)-aryl.

3. Complex according to either of Claims 1 and 2,
wherein R⁵, R⁶, R⁷, R⁸ are -Ph.

4. Complex according to any of Claims 1 to 3,
wherein R⁹ and R¹⁰ are -H.

5. Complex according to any of Claims 1 to 4,
wherein at least one of the radicals R¹, R⁴ is -H.

6. Complex according to any of Claims 1 to 5,
wherein R² and R³ are -(C₁-C₁₂)-alkyl.

7. Complex according to any of Claims 1 to 6,
wherein the ligand corresponding to formula (I) has the structure (1) or (2):

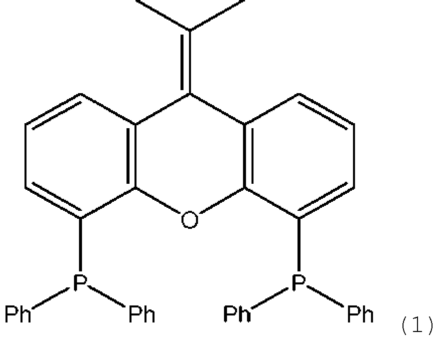

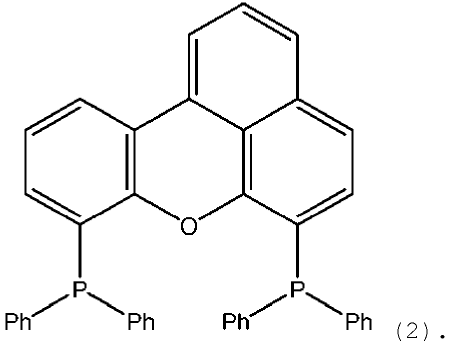

8. Complex according to any of Claims 1 to 7,
wherein the ligand corresponding to formula (I) has the structure (1):

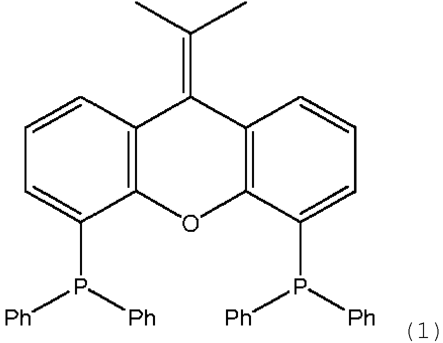

9. Complex according to any of Claims 1 to 7,
wherein the ligand corresponding to formula (I) has the structure (2):

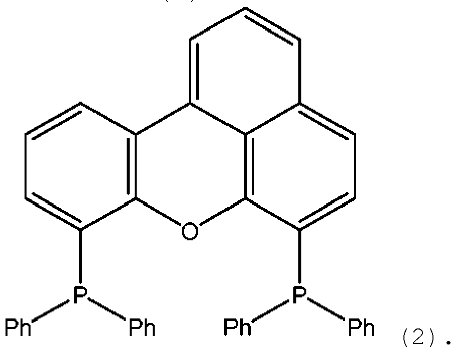

10. Complex according to any of Claims 1 to 9,
wherein the complex has exactly one ligand corresponding to formula (I).

11. Complex according to any of Claims 1 to 10,
wherein the complex has at least two iodine ligands.

12. Complex according to Claim 11,
wherein the complex has exactly two iodine ligands.

13. Complex according to any of Claims 1 to 10, wherein the complex has at least two bromine ligands.

14. Complex according to Claim 13,
wherein the complex has exactly two bromine ligands.

15. Use of a complex according to any of Claims 1 to 14 for catalysis of a hydroformylation reaction.

## Revendications

1. Complexe comprenant :
a) du Pt;
b) un ligand selon la formule (I) : 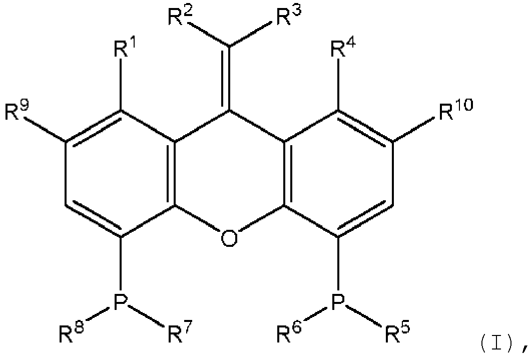 dans laquelle R¹, R², R³, R⁴, R⁵, R⁶, R⁷, R⁸, R⁹, R¹⁰ sont choisis parmi : -H, -(C₁-C₁₂)-alkyle, -(C₆-C₂₀)-aryle et R¹, R², R³, R⁴ peuvent former des systèmes cycliques condensés ;
c) un ligand iode ou un ligand brome.

2. Complexe selon la revendication 1,
R⁵, R⁶, R⁷, R⁸ représentant -(C₆-C₂₀)-aryle.

3. Complexe selon l'une des revendications 1 ou 2,
R⁵, R⁶, R⁷, R⁸ représentant -Ph.

4. Complexe selon l'une des revendications 1 à 3,
R⁹ et R¹⁰ représentant -H.

5. Complexe selon l'une des revendications 1 à 4,
au moins l'un des radicaux R¹, R⁴ représentant -H.

6. Complexe selon l'une des revendications 1 à 5,
R² et R³ représentant -(C₁-C₁₂)-alkyle.

7. Complexe selon l'une des revendications 1 à 6,
le ligand selon la formule (I) présentant la structure (1) ou (2) :

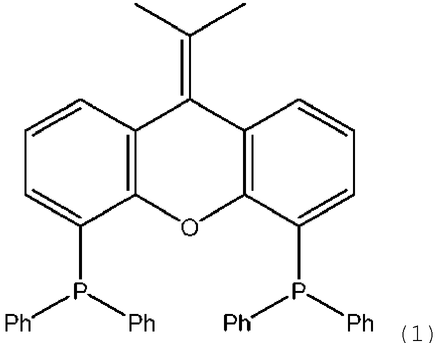

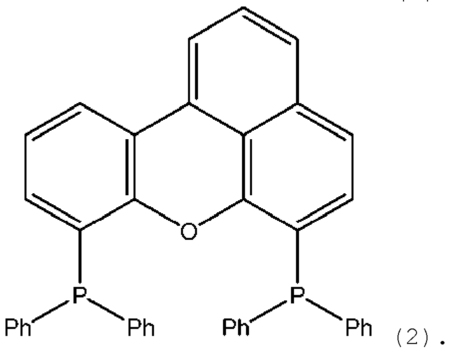

8. Complexe selon l'une des revendications 1 à 7,
le ligand selon la formule (I) présentant la structure (1) :

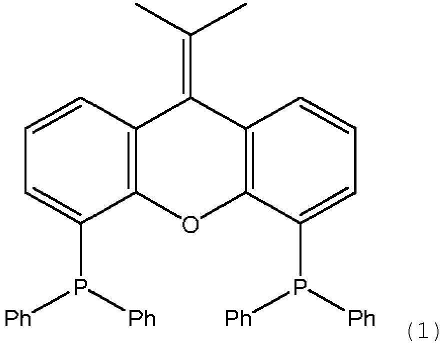

9. Complexe selon l'une des revendications 1 à 7,
le ligand selon la formule (I) présentant la structure (2) :

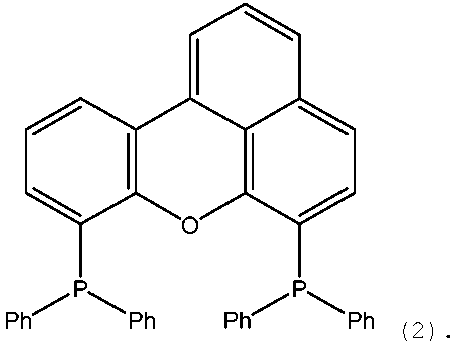

10. Complexe selon l'une des revendications 1 à 9,
le complexe présentant exactement un ligand selon la formule (I).

11. Complexe selon l'une des revendications 1 à 10,
le complexe présentant au moins deux ligands iode.

12. Complexe selon la revendication 11,
le complexe présentant exactement deux ligands iode.

13. Complexe selon l'une des revendications 1 à 10,
le complexe présentant au moins deux ligands brome.

14. Complexe selon la revendication 13,
le complexe présentant exactement deux ligands brome.

15. Utilisation d'un complexe selon l'une des revendications 1 à 14 pour la catalyse d'une réaction d'hydroformylation.
